# EUROPEAN PATENT APPLICATION

(11) **EP 4 516 398 A1**
(43) Date of publication of application: **05.03.2025**
(21) Application number: 23795017.5
(22) Date of filing: 12.04.2023
(51) Int. Cl.: B01J 31/06

(54) **EGGSHELL-TYPE CATALYST, PREPARATION METHOD THEREFOR, AND USE THEREOF IN PROPYLENE HYDROFORMYLATION REACTION**

(30) Priority: 24.04.2022 CN 202210435228
(71) Applicant: Dalian Institute Of Chemical Physics, Chinese Academy of Sciences, Liaoning 116000 (CN)
(72) Inventor: LI, Cunyao, Dalian, Liaoning 116000 (CN); YAN, Li, Dalian, Liaoning 116000 (CN); DING, Yunjie, Dalian, Liaoning 116000 (CN); DING, Yulong, Dalian, Liaoning 116000 (CN); JIANG, Miao, Dalian, Liaoning 116000 (CN); MA, Lei, Dalian, Liaoning 116000 (CN); JI, Guangjun, Dalian, Liaoning 116000 (CN)
(74) Representative: ZHAOffice SPRL
(86) International application number: PCT/CN2023/087737
(87) International publication number: WO 2023/207584

(57) **Abstract**

The present invention discloses an eggshell type catalyst, and a preparation method and application thereof in propylene hydroformylation reaction. The eggshell type catalyst uses phosphine ligand resin pellets with developed pore structure as a carrier and one, two or more than two of metals Rh, Co, Ir, Ru and Pd as active components. The spatial distribution of the active metal components in the resin pellets is effectively regulated through a method of finely regulating the porous channel structure of the carrier resin pellets and adding competitive adsorption and coordination, so that the prepared eggshell type catalyst has the characteristics of high activity in the propylene hydroformylation reaction and good selectivity of n-butyraldehyde in the product aldehyde.

## Description

### Technical Field

The present invention belongs to the field of heterogeneous catalysis, and particularly relates to an eggshell type catalyst, and a preparation method and application thereof in propylene hydroformylation reaction.

### Background

The preparation of butyraldehyde by propylene hydroformylation reaction is a very important process in industry. Propylene can be derived from petroleum cracking, coal chemical industry, etc., and product butyraldehyde, especially n-butyraldehyde, is an important chemical raw material. At present, the propylene hydroformylation reaction mainly uses a rhodium complex as a catalyst, including two technologies of homogeneous catalysis and biphasic catalysis.

The homogeneous technology is successfully developed jointly by Union Carbide Corporation, Davy Company and Johnson Matthey Company (Chemical Technology Market, 2005, 8:15-18). The name of the catalyst commodity used in this method is ROPAC, which is generally carbonyltris(triphenylphosphine)rhodium(I) hydride, acetylacetonatocarbonyltriphenylp hosphinerhodium(I) and rhodium (triphenylphosphine)carbonylacetylacetonate ligand. The biphasic technology was industrially used in 1984, and used a Wilkinson type complex HRh(CO)(TPPTS)₃ of TPPTS and Rh as a catalyst.

The homogeneous technology and the biphasic technology are difficult to recover Rh catalysts of noble metal. Although the biphasic technology greatly simplifies the separation and recovery steps of noble metal catalysts (J Mol Catal A: Chem, 1995, 97: 65-72), the catalyst with TPPTS as the phosphine ligand is more prone to inactivation (J Mol Catal A: Chem, 1997, 126: 133-140), and the inactivation process is shown in formula 1.

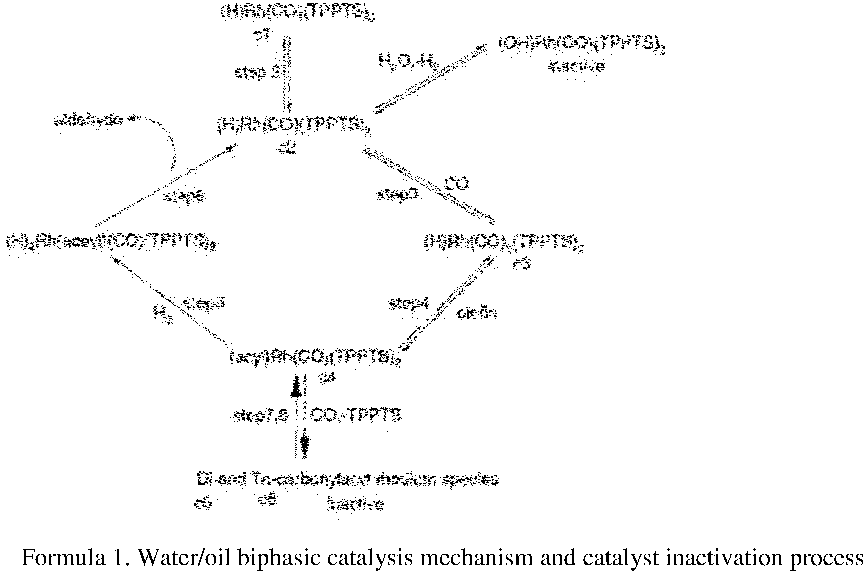

At present, many methods to achieve the polyphase propylene hydroformylation reaction have been reported in the literature. These methods have achieved certain success, but the loss of catalyst active components, easy inactivation of the catalyst and low selectivity of product aldehyde are still problems that need to be solved. There are no industrial examples of this type of catalyst (Eur. J. Org. Chem., 2012, 2012: 6309-6320).

### Summary

To solve the above problems, the purpose of the present invention is to provide an eggshell type catalyst for the hydroformylation reaction of olefin and a preparation method and application thereof.

The technical solution of the present invention is as follows:
An eggshell type catalyst is provided. Active components are selected from one, two or more than two of Rh, Co, Ir, Ru and Pd; a carrier of the eggshell type catalyst is a phosphine ligand resin pellet;

The phosphine ligand resin pellet is formed by autopolymerization of one or more than one of monodentate ligands of vinyl or copolymerization of monodentate ligands of vinyl and polydentate ligands of vinyl; the particle size of the resin pellet ranges from 0.5-7 mm (preferably 0.5-4 mm); metal active components are coordinated on a surface layer of the polymer pellet to form a catalyst eggshell layer; and the thickness (the depth of the metal activity from a carrier surface coordinated to the carrier) of the catalyst eggshell layer (a surface layer of the carrier containing the metal active components is called the eggshell layer) is 0.1-0.2 mm (preferably 0.1-0.15 mm).

The range of metal load of the eggshell type catalyst is 0.01-8 wt%, and a preferred range is 0.1-2 wt%;

The preparation of the eggshell type catalyst uses a method of excessive solution impregnation; the phosphine ligand resin pellet is added to a solution containing one or more than one of precursors of the active components Rh, Co, Ir, Ru and Pd, stirred fully and coordinated; and active metal of the eggshell type catalyst is coordinated with the phosphine ligand in a resin skeleton to form the catalyst eggshell layer, thereby obtaining the eggshell type catalyst.

The monodentate phosphine ligands of vinyl are one or more than one of the following:

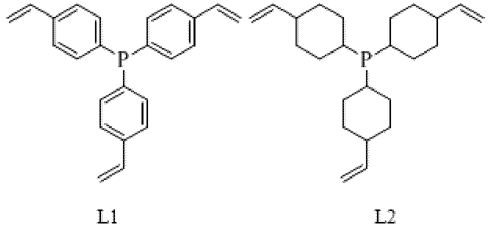

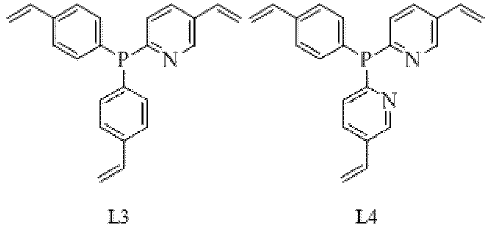

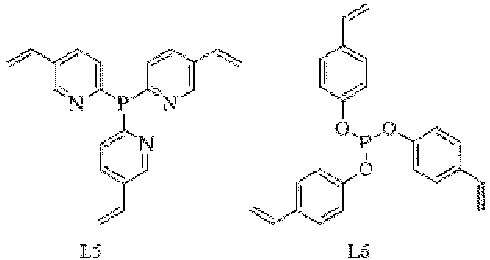

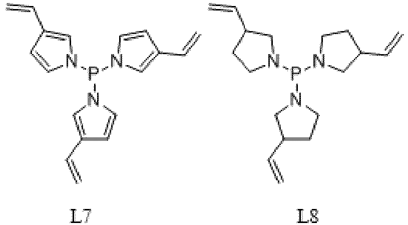

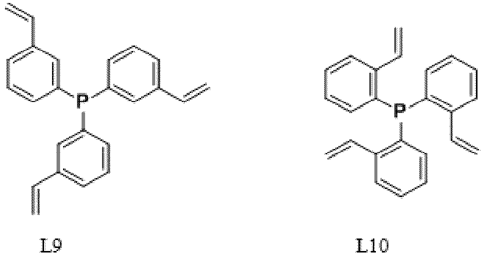

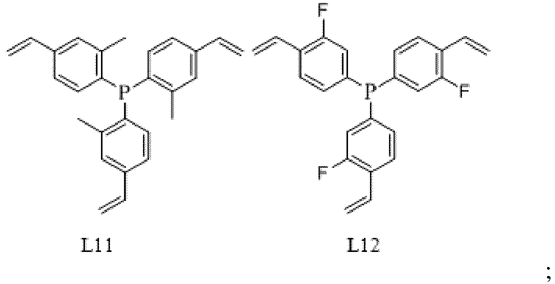

The polydentate ligands of vinyl are one or more than one of the following:

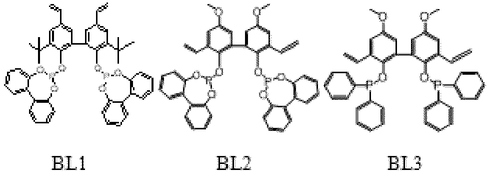

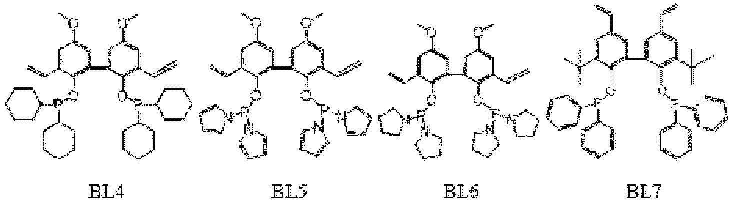

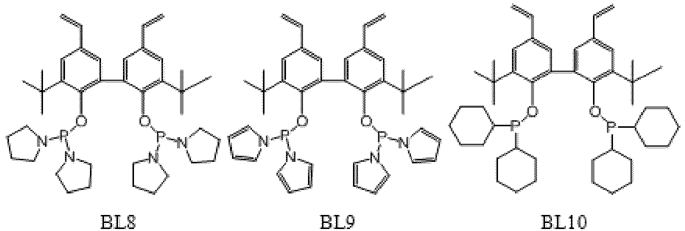

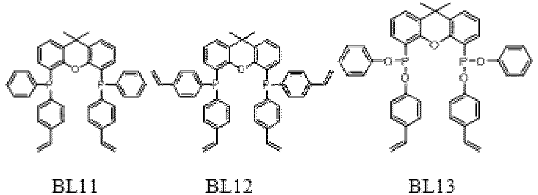

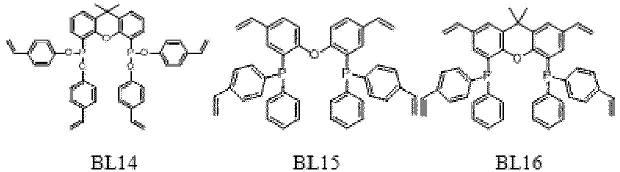

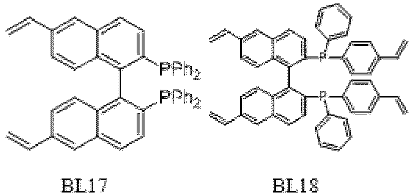

The specific surface area of the phosphine ligand resin pellet is 50-3000 m²/g, and a preferred range is 200-1500 m²/g; the pore volume is 0.2-10.0 cm³/g, and preferably 0.5-3.0 cm³/g; and the pore size is distributed in the range of 0.01-100.0 nm, and preferably 0.2-10.0 nm.

A preparation method of the phosphine ligand resin pellet comprises:
after fully dissolving one or more than one of monodentate phosphine ligands of vinyl, adding or not adding polydentate phosphine ligands of vinyl, adding a free radical initiator and preparing the phosphine ligand resin pellet by suspension polymerization.

The preparation method of the eggshell type catalyst comprises: firstly, adding the phosphine ligand resin pellet to the solution containing a competitive coordination agent and then removing a solvent; then adding the phosphine ligand resin pellet to a precursor impregnation solution containing active metal components; fully stirring to coordinate the active metal components with the phosphine ligand in the surface layer of the resin pellet; and evaporating the solvent to obtain the eggshell type catalyst.

Specific preparation steps of the eggshell type catalyst are:
a) under the protection of inert atmosphere gas of 273-493 K (preferably 273-453 K), adding the monodentate phosphine ligands of vinyl to the solvent, adding or not adding polydentate phosphine ligands of vinyl, and stirring evenly for use;
b) under the protection of inert atmosphere gas of 298-433 K (preferably 298-393 K), adding a suspending agent to water to fully dissolve; adding the above mixed solution to an aqueous phase while stirring; adding the free radical initiator; and polymerizing the phosphine ligand into a resin pellet at a stirring speed of 20-2000 r/min (preferably 50-800 r/min);
c) taking out solid particles obtained in step b); cleaning by a solvent of 10-1000 (preferably 20-800) times the volume; and then removing the solvent at 298-453 K (preferably 298-393 K) to obtain the phosphine ligand resin pellet;
d) under the protection of inert atmosphere gas of 273-453 K (preferably 273-413 K), stirring the phosphine ligand resin pellet obtained in step c) in a solution containing competitive adsorption and coordination for 0.1-10 hours (preferably 0.1-5 hours); then removing the solvent at 298-433 K (preferably 298-393 K); then adding the resin pellet to a solution containing the precursors of the active metal components; stirring for 0.1-100 hours, and preferably for a range of 0.5-12 hours; and next, removing the solvent at 273-433 K (preferably 273-393 K) to obtain the eggshell type catalyst, wherein the concentration range of the competitive adsorption and coordination and the active metal in the precursor solution is 0.0001-5 mol•L⁻¹ respectively (preferably 0.0001-1 mol•L⁻¹).

An organic solvent in step a) is selected from one or more than one of toluene, xylene, benzene, n-alkane of C₅-C₁₅ and cyclohexane.

Before the resin pellet is polymerized, the concentration range of the monophosphine ligands of vinyl in the solvent is 0.01-1000 g/L, preferably 1-100 g/L; a molar ratio of the monophosphine ligands to the polydentate ligands is 5:1-200:1 when the monophosphine ligands of vinyl and the polydentate ligands of vinyl are added at the same time; a molar ratio of the monodentate phosphine ligands containing alkylene to the free radical initiator is 500:1-10:1, preferably 100:1-10:1.

The free radical initiator in step b) is one or more than one of dibenzoyl peroxide, cyclohexanone peroxide, tert-butyl hydroperoxide or azodiisobutyronitrile; the suspending agent is selected from one or more than one of N-dodecyl dimethylamine, sodium stearate, calcium dodecylbenzene sulfonate, polyvinyl alcohol and polyvinylpyrrolidone; and a volume ratio of oil phase and aqueous phase in suspension polymerization is 1000:1-1:1000 (preferably 100:1-1:1000).

The solvent for cleaning the resin pellet in step c) is one or more than one of benzene, toluene, xylene, isopropanol, ethanol, dichloromethane, petroleum ether, trichloromethane, water or tetrahydrofuran.

The competitive adsorbent and coordination agent in step d) is selected from one or more than one of MgCl₂, MgSO₄, AlCl₃, CaCl₂, CuSO₄ and ZnSO₄; the impregnation of a competitive adsorbent and the active metal uses a method of excessive solution impregnation; and a volume ratio of the resin pellet to an impregnation solution is 1:1.01-1:1000 (preferably 1:1.01-1:500).

The inert atmosphere gas in steps a), b) and d) are selected from one or more than one of CO₂, Ar, He and N₂.

The active components are one or more than one of Rh, Co, Ir, Ru and Pd, wherein the precursor of Rh is one or more than one of RhH(CO)(PPh₃)₃, Rh(CO)₂(acac), RhCl₃ and Rh(CH₃COO)₂; the precursor of Co is one or more than one of Co(CH₃COO)₂, Co(CO)₂(acac), Co(acac)₂ and CoCl₂; the precursor of Ir is one or more than one of Ir(CO)₃(acac), Ir(CH₃COO)₃, Ir(acac)₃ and IrCl₄; the precursors of Ru are dichloride (1,5-cyclooctadiene) ruthenium (II), RuCl₃, Ru(acac)₃, triruthenium dodecacarbonyl, [RuAr₂(benzene)]₂, [RuAr₂(p-cymene)]₂, [RuAr₂(mesitylene)]₂, [(*π*-ally)Ru(cod)]₂ and [(π-ally)Ru(nbd)]₂; the precursor of Pd is one or more than one of Pd(CH₃COO)₂, Pd(acac)₂, PdCl₂, Pd(PPh₃)₄ and PdCl₂(CH₃CN)₂, and the range of the metal load in the catalyst is 0.01-8 wt%, preferably 0.1-2 wt%.

An application of the eggshell type catalyst in propylene hydroformylation reaction is provided, comprising a reaction process of loading an eggshell type into a reactor and introducing raw material propylene and mixed gas containing H₂ and CO for propylene hydroformylation reaction.

The main components of the mixed gas are H₂ and CO; the volume content of H₂+CO is 10-100% (preferably 30-100%); a volume ratio of H₂/CO is 0.1-10.0 (preferably 0.6-1.5); other gas components are one or more than one of CO₂, N₂, He and Ar; the purity of the raw propylene is 10-100% (preferably 50-100%), and other gas components are one or more than one of CO₂, N₂, He and Ar; reaction temperature is 313-433 K (preferably 343-393 K); reaction pressure is 0.1-6.0 MPa (preferably 0.3-2.0 MPa); airspeed of the mixed gas is 100-10000 h⁻¹ (preferably 300-3000 h⁻¹); and propylene airspeed is 0.01-40.0 h⁻¹ (preferably 0.1-10.0).

The reactor is a slurry bed, a trickle bed, a tank reactor, a radial reactor or a tubular reactor, preferably the slurry bed reactor; the eggshell type catalyst has high activity; the proportion of n-butyraldehyde in the product aldehyde is high; and this type of eggshell type catalyst exhibits a strong prospect of industrial application.

The reaction principle of the present invention is as follows:
The eggshell type catalyst disclosed by the present invention uses the phosphine ligand resin pellet with developed pore structure as the carrier, and the metal active components are coordinated in the eggshell layer of the resin pellet containing high-concentration phosphine ligands and supported, so as to avoid the loss of the active components and the ligands. At the same time, in catalyst preparation, the distribution of the active metal components in the resin pellet can be effectively regulated by adding the competitive coordination agent and other means. The specific distribution state of the active metal components and the confinement effect of porous channels of the resin pellet make the eggshell type catalyst have high selectivity of n-butyraldehyde when applied to the propylene hydroformylation reaction.

The present invention has the following beneficial effects:
The active metal components of the eggshell type catalyst provided by the present invention are firmly fixed on the surface layer of the resin pellet to realize the heterogenizing of the catalyst of the propylene hydroformylation reaction. By strictly controlling the preparation steps of the eggshell type catalyst, the activity of the catalyst is high in the propylene hydroformylation reaction, and the selectivity of n-butyraldehyde is good. The eggshell type catalyst has good mass and heat transfer effect and high utilization rate of active center, and is suitable for the industrial application of the propylene hydroformylation reaction.

### Description of Drawings

Fig. 1 is an N₂ physical adsorption curve of a resin pellet obtained in embodiment 1;
Fig. 2 is a pore size distribution diagram of a resin pellet obtained in embodiment 1.

### Detailed Description

The following embodiments better illustrate the present invention, but do not limit the protection scope of the present invention.

### Embodiment 1

Preparation method of phosphine ligand resin pellet:
In a CO₂ atmosphere at 20°C, 5 g of tri(3-vinylphenyl) phenylphosphine (ligand L9) is weighed, dissolved in 70 ml of cyclohexane, and stirred evenly for use. In a CO₂ atmosphere at 40°C, 0.5 g of sodium stearate (suspending agent) is dissolved in 20 mL of deionized water; the solution in which phosphine ligands are dissolved is added to the above deionized water in which the suspending agent is dissolved while stirred (mechanical stirring, at stirring speed of 180 r/min); 0.1 g of azodiisobutyronitrile (initiator) is added, and then continuously stirred (mechanical stirring, at stirring speed of 180 r/min) at 90°C and polymerized for 12 hours.

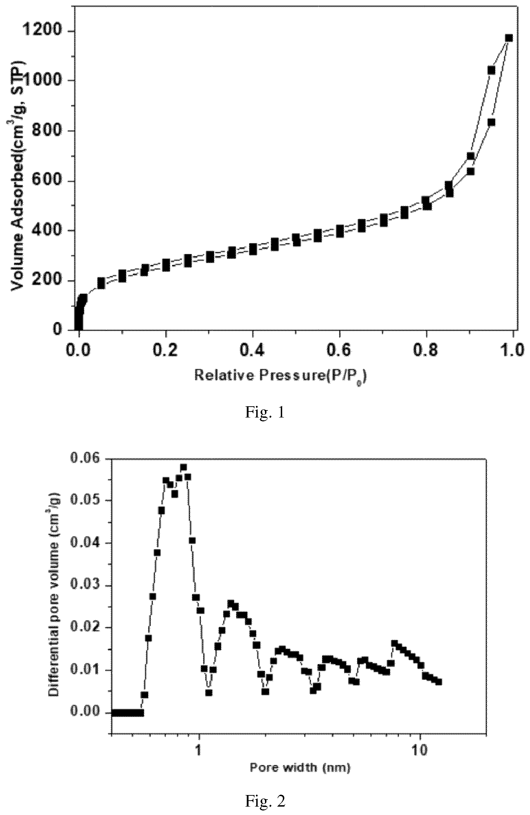

After cooling to room temperature, 400 mL of resin pellets are filtered out, washed by tetrahydrofuran, and then dried under vacuum at 75°C for 10 hours to obtain phosphine ligand resin pellets with a diameter range of 0.5 mm-2.5 mm and an average particle size of 1.8 mm. The N₂ physical adsorption curve of the resin pellets is shown in Fig. 1. It is calculated that the pore volume of the polymer pellets is 1.82 cm³/g and the specific surface area is 908 m²/g. The pores of the resin pellets are mainly distributed at 0.4-10 nm (Fig. 2, a pore size distribution curve of phosphine ligand resin pellets).

Preparation method of eggshell type catalyst:
The resin pellets of 1.5-2.0 mm are screened. 1 g of resin pellets of 1.5-2.0 mm are weighed in a CO₂ atmosphere at 30°C, added to 20 mL of 0.6 mol•L⁻¹ MgSO₄ aqueous solution, and stirred for 1 hour. The filtered resin pellets are dried under vacuum at 80°C for 10 hours.

In a CO₂ atmosphere at 35°C, 1 g of resin pellets adsorbed with competitive adsorbents are then added to 50.0 mL of xylene containing 5.1 mg of Rh(CO)₂(acac) (CAS No. 14874-82-9), stirred at 45°C and coordinated for 48 hours.

The solvent is removed under vacuum at 45°C, to obtain the eggshell type catalyst applied to the hydroformylation reaction of olefin. The cross section is observed by an electron microscope. The active component Rh is uniformly coordinated on the surface layer of the polymer pellets. The thickness of the catalyst layer (shell layer) containing metal Rh is 0.15 mm. The distribution of Rh in the eggshell layer is uniform. The measured metal load of the catalyst is 0.20%.

### Embodiment 2

In embodiment 2, when the phosphine ligand resin pellets are prepared, the synthesis process and conditions of the resin pellets and the eggshell type catalyst are the same as those of embodiment 1, except that 5 g of phosphine ligands L9 is replaced with 2.5 g of phosphine ligands L1 and 2.5 g of phosphine ligand L10. The cross section is observed by an electron microscope. The active component Rh is uniformly coordinated on the surface layer of the polymer pellets. The thickness of the catalyst layer (shell layer) containing metal Rh is 0.15 mm. The distribution of Rh in the eggshell layer is uniform. The measured metal load of the catalyst is 0.20%.

### Embodiment 3

In embodiment 3, when the phosphine ligand resin pellets are prepared, the synthesis process and conditions of the resin pellets and the eggshell type catalyst are the same as those of embodiment 1, except that 5 g of phosphine ligands L9 is replaced with 5 g of phosphine ligands L6. The cross section is observed by an electron microscope. The active component Rh is uniformly coordinated on the surface layer of the polymer pellets. The thickness of the catalyst layer (shell layer) containing metal Rh is 0.15 mm. The distribution of Rh in the eggshell layer is uniform. The measured metal load of the catalyst is 0.20%.

### Embodiment 4

In embodiment 4, when the phosphine ligand resin pellets are prepared, the synthesis process and conditions of the resin pellets and the eggshell type catalyst are the same as those of embodiment 1, except that 5 g of phosphine ligands L9 is replaced with 5 g of phosphine ligands L11. The cross section is observed by an electron microscope. The active component Rh is uniformly coordinated on the surface layer of the polymer pellets. The thickness of the catalyst layer (shell layer) containing metal Rh is 0.15 mm. The distribution of Rh in the eggshell layer is uniform. The measured metal load of the catalyst is 0.20%.

### Embodiment 5

In embodiment 5, when the phosphine ligand resin pellets are prepared, the synthesis process and conditions of the resin pellets and the eggshell type catalyst are the same as those of embodiment 1, except that 5 g of phosphine ligands L9 is replaced with 4.8 g of phosphine ligands L9 and 0.2 g of phosphine ligand BL2. The cross section is observed by an electron microscope. The active component Rh is uniformly coordinated on the surface layer of the polymer pellets. The thickness of the catalyst layer (shell layer) containing metal Rh is 0.15 mm. The distribution of Rh in the eggshell layer is uniform. The measured metal load of the catalyst is 0.20%.

### Embodiment 6

In embodiment 6, when the phosphine ligand resin pellets are prepared, the synthesis process and conditions of the resin pellets and the eggshell type catalyst are the same as those of embodiment 1, except that 5 g of phosphine ligands L9 is replaced with 4.5 g of phosphine ligands L9 and 0.5 g of phosphine ligand BL17. The cross section is observed by an electron microscope. The active component Rh is uniformly coordinated on the surface layer of the polymer pellets. The thickness of the catalyst layer (shell layer) containing metal Rh is 0.15 mm. The distribution of Rh in the eggshell layer is uniform. The measured metal load of the catalyst is 0.20%.

### Embodiment 7

In embodiment 7, when the eggshell type catalyst is prepared, the synthesis process and conditions of the resin pellets and the eggshell type catalyst are the same as those of embodiment 1, except that 0.6 mol•L⁻¹ MgSO₄ aqueous solution is replaced with 1.0 mol•L⁻¹ CaCl₂ aqueous solution, when a competitive adsorbent is impregnated. The cross section is observed by an electron microscope. The active component Rh is uniformly coordinated on the surface layer of the polymer pellets. The thickness of the catalyst layer (shell layer) containing metal Rh is 0.15 mm. The distribution of Rh in the eggshell layer is uniform. The measured metal load of the catalyst is 0.20%.

### Embodiment 8

In embodiment 8, the synthesis process and conditions of the resin pellets and the eggshell type catalyst are the same as those of embodiment 1, except that Rh(CO)₂(acac) is replaced with cobalt octacarbonyl of the same molar number. An eggshell type Co-based catalyst can be obtained, and the thickness of an eggshell layer is 0.13 mm.

### Embodiment 9

In embodiment 9, the synthesis process and conditions of the resin pellets and the eggshell type catalyst are the same as those of embodiment 1, except that Rh(CO)₂(acac)is replaced with IrCl4 of the same molar number. An eggshell type Ir-based catalyst can be obtained, and the thickness of a catalyst layer (shell layer) is 0.17 mm.

### Embodiment 10

In embodiment 10, the synthesis process and conditions of the resin pellets and the eggshell type catalyst are the same as those of embodiment 1, except that Rh(CO)₂(acac) is replaced with [(π-ally)Ru(cod)]₂ of the same molar number. The thickness of a catalyst layer (shell layer) is 0.14 mm.

### Embodiment 11

In embodiment 11, in the step of impregnating the active metal, the synthesis process and conditions of the resin pellets and the eggshell type catalyst are the same as those of embodiment 1, except that 50.0 mL of xylene is replaced with 100 mL of 2-methyltetrahydrofuran for preparing the impregnation solution. The thickness of a catalyst layer (shell layer) is 0.17 mm.

### Reference example 1

In reference example 1, the type and the use amount of the metal Rh precursor in the impregnation process are the same as those of embodiment 1 except that a homogeneous catalyst is prepared by the solvothermal polymerization method mentioned in the previous patent CN104707660B.

A specific implementation method is as follows: 50 g of phosphine ligands L9 and 1 g of azodiisobutyronitrile are dissolved in 500 ml of tetrahydrofuran in a 1000 ml enamel high-pressure reactor, stirred for 2 hours, and immobilizd under protection of 373K nitrogen for 24 hours to perform polymerization reaction. Then, the solution is cooled to room temperature, and the solvent is removed at room temperature to obtain a powdered polymer of the phosphine ligand.

5.1 mg of Rh(CO)₂(acac) is dissolved in 30.0 mL of tetrahydrofuran under the protection atmosphere of 298K and inert gas. 1.0 g of powdered polymer carrier of phosphine ligand (crushed to 200-300 meshes) is added. The mixture is stirred under the protection atmosphere of 298K and inert gas for 24 hours. Then, the solvent is vacuumed away at room temperature, to obtain a homogeneous powdered catalyst for the hydroformylation reaction.

### Reference example 2

In reference example 2, the synthesis process and conditions of the resin pellets and the eggshell type catalyst are the same as those of embodiment 1, except that there is no step of impregnating the competitive adsorbent. The cross section is observed by an electron microscope. The active component Rh is uniformly coordinated on the surface layer of the polymer pellets. The thickness of the catalyst layer (shell layer) containing metal Rh is 0.04 mm. The distribution of Rh in the eggshell layer is uniform. The measured metal load of the catalyst is 0.20%.

### Reference example 3

In reference example 3, the synthesis process and conditions of the resin pellets and the eggshell type catalyst are the same as those of embodiment 1, except that there is no step of impregnating the competitive adsorbent and 50.0 mL of xylene is replaced with 5.0 mL of xylene for preparing the impregnation solution. The cross section is observed by an electron microscope. The active component Rh is uniformly coordinated on the surface layer of the polymer pellets. The thickness of the catalyst layer (shell layer) containing metal Rh is 0.02 mm. The distribution of Rh in the eggshell layer is uniform. The measured metal load of the catalyst is 0.20%.

### Reference example 4

In reference example 4, the preparation steps of the eggshell type catalyst are changed to:
1 g of resin pellets of 1.5-2.0 mm are weighed in a CO₂ atmosphere at 30°C, added to 20 mL of 0.6 mol•L⁻¹ MgSO₄ aqueous solution, and stirred for 1 hour. The filtered resin pellets are dried under vacuum at 80°C for 10 hours.
1 g of resin pellets adsorbed with competitive adsorbents are added to 500.0 mL of xylene containing 5.1 mg of Rh(CO)₂(acac) (CAS No. 14874-82-9) at -10°C, stirred at -10°C and coordinated for 48 hours.

The solvent is removed under vacuum at 45°C, to obtain the eggshell type catalyst applied to the hydroformylation reaction of olefin. The cross section is observed by an electron microscope. The active component Rh is uniformly distributed on the surface layer of the polymer pellets. The thickness of the catalyst layer (shell layer) containing metal Rh is 0.22 mm, and the distribution of Rh in the eggshell layer is uniform. The measured metal load of the catalyst is 0.20%.

### Reference example 5

In reference example 5, when the resin pellets are prepared, the synthesis process of the resin pellets is the same as that of embodiment 1, except that 5 g of ligands L9 is replaced with 5 g of monodentate phosphine ligands of vinyl

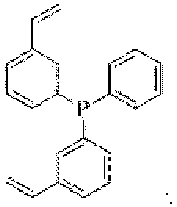

The preparation steps of the eggshell type catalyst are changed to:
1 g of resin pellets of 1.5-2.0 mm are weighed in a CO₂ atmosphere at 70°C, added to 20 mL of 0.6 mol•L⁻¹ MgSO₄ aqueous solution, and stirred for 1 hour. The filtered resin pellets are dried under vacuum at 80°C for 10 hours.
1 g of resin pellets adsorbed with competitive adsorbents are added to 500.0 mL of xylene containing 5.1 mg of Rh(CO)₂(acac) (CAS No. 14874-82-9) at -10°C, stirred at -10°C and coordinated for 48 hours.

The solvent is removed under vacuum at 45°C, to obtain the eggshell type catalyst applied to the hydroformylation reaction of olefin. The cross section is observed by an electron microscope. The active component Rh is uniformly distributed on the surface layer of the polymer pellets. The thickness of the catalyst layer (shell layer) containing metal Rh is 0.27 mm, and the distribution of Rh in the eggshell layer is uniform. The measured metal load of the catalyst is 0.20%.

### Reference example 6

In reference example 6, when the eggshell type catalyst is prepared, the active metal is not impregnated, and only the competitive adsorbent is impregnated. The synthesis process and conditions of the resin pellets and the eggshell type catalyst are the same as those of embodiment 1. Specific steps are as follows:
Preparation of eggshell type catalyst:
The resin pellets of 1.5-2.0 mm are screened. 1 g of resin pellets of 1.5-2.0 mm are weighed in a CO₂ atmosphere at 30°C, added to 20 mL of mixed aqueous solution of 0.6 mol•L⁻¹ MgSO₄, 0.6 mol•L⁻¹ MgCl₂, 0.6 mol•L⁻¹ MgSO₄, 0.6 mol•L⁻¹ AlCl₃, 0.6 mol•L⁻¹ CaCl₂, 0.6 mol•L⁻¹ CuSO₄ and 0.6 mol•L⁻¹ ZnSO₄, and stirred for 1 hour. The filtered resin pellets are dried under vacuum at 80°C for 10 hours.

### Embodiment 12

1 g of the catalysts prepared in embodiments 1-11 and reference examples 1-6 are loaded into a slurry bed reactor with a capacity of 100 ml, and 60 ml of valeraldehyde is added as slurry liquid. The reaction mixed gas (H₂:CO:C₃H₆=1:1:1) is introduced. The hydroformylation reaction is carried out at 378K, 0.8 MPa, an airspeed of the reaction mixed gas of 1000 h⁻¹ and a stirring rate of 600 RPM. In the reaction, a collection tank at constant temperature of -10°C is used for collection. The reaction products and the slurry liquid carried by tail gas are all dissolved in the collection tank. The collected liquid is analyzed by HP-7890N gas chromatography with HP-5 capillary column and FID, and ethanol is used as the internal standard. The tail gas is analyzed online by HP-7890N gas chromatography with Porapak-QS column and TCD. Reaction results are listed in Table 1.

**Table 1. Performance of Propylene Hydroformylation of Slurry Bed of Catalyst in Embodiment 1- Embodiment 11 and Reference Examples**

| Entry | Catalysts | S_{BET}/(m^{2/} g) | Pore Size Distribution (nm) | Propylene TOF(h⁻¹) | Aldehyde Selectivity /% | n-butyraldehyde: isobutyraldehyde |
|---|---|---|---|---|---|---|
| 1 | Embodiment 1 | 902 | 0.2-10.0 | 733.6 | 99.0 | 8.7 |
| 2 | Embodiment 2 | 873 | 0.1-8.0 | 744.5 | 98.7 | 10.9 |
| 3 | Embodiment 3 | 794 | 0.3-9.0 | 953.2 | 99.0 | 8.0 |
| 4 | Embodiment 4 | 705 | 0.1-8.0 | 719.0 | 99.0 | 9.8 |
| 5 | Embodiment 5 | 897 | 0.1-10.0 | 1236.7 | 98.8 | 29.7 |
| 6 | Embodiment 6 | 840 | 0.1-9.0 | 724.4 | 98.9 | 33.2 |
| 7 | Embodiment 7 | 903 | 0.2-10.0 | 716.3 | 98.7 | 8.9 |
| 8 | Embodiment 8 | 904 | 0.2-10.0 | 111.6 | 97.4 | 8.4 |
| 9 | Embodiment 9 | 902 | 0.2-10.0 | 63.3 | 98.0 | 8.1 |
| 10 | Embodiment 10 | 903 | 0.2-10.0 | 74.2 | 97.4 | 8.0 |
| 11 | Embodiment 11 | 902 | 0.2-10.0 | 736.3 | 98.8 | 9.0 |
| 12 | Reference example 1 | 1131 | 0.1-5.0 | 605.7 | 97.3 | 8.3 |
| 13 | Reference example 2 | 905 | 0.2-10.0 | 739.3 | 98.7 | 2.6 |
| 14 | Reference example 3 | 903 | 0.2-10.0 | 636.4 | 98.6 | 2.5 |
| 15 | Reference example 4 | 899 | 0.2-10.0 | 653.1 | 98.9 | 8.7 |
| 16 | Reference example 5 | 549 | 0.2-4.0 | 606.2 | 99.1 | 8.9 |
| 17 | Reference example 6 | 902 | 0.2-10.0 | 0 | -- | -- |

It can be seen from Table 1 that embodiments 1-11 are the eggshell type catalyst. The ratio of n-butyraldehyde to isobutyraldehyde in the product aldehyde can be basically above 8.0, and the value of propylene TOF can be above 700 h⁻¹. When the copolymerization solution of monophosphine ligands and polydentate phosphine ligands is adopted to prepare resin pellets to prepare the eggshell type catalyst, the ratio of n-butyraldehyde to isobutyraldehyde in the product butyaldehyde can be about 30 (embodiments 5 and 6). In embodiments 8, 9 and 10, the active metals are Co, Ir and Ru, respectively, and have lower reaction performance than the catalysts with the active metal of Rh (embodiment 1) (however, Co, Ir, Ru, etc. have lower price and are more suitable for non-Rh metals in some industrial occasions). When the active center is Rh, it can be seen that the activity of the propylene hydroformylation reaction of the eggshell type catalyst is higher than that of the homogeneous catalyst of the corresponding reference example (reference example 1) and the selectivity of n-butyraldehyde is similar. When there is no step of impregnating the competitive adsorbent (reference example 2), the ratio of n-butyraldehyde to isobutyraldehyde in the eggshell type catalyst is 2.6. The impregnation of the competitive adsorbent can improve the spatial distribution of the active components of the catalyst in the resin pellets, so that the selectivity of n-butyraldehyde is higher in the propylene hydroformylation reaction. The eggshell layer (0.02 mm) of the catalyst prepared by reference example 3 is thinner than the eggshell layers of the catalysts prepared by embodiments 1-11. Thus, the selectivity of the product n-butyraldehyde is significantly lower than that of embodiment 1 and the activity is also lower than that of embodiment 1. The thicknesses of the eggshell layers prepared by reference example 4 and reference example 5 are 0.22 mm and 0.27 mm, respectively, which are thicker than that of embodiment 1. The selectivity of n-butyraldehyde is not significantly improved compared with embodiment 1, and the reactivity of propylene is lower than that of embodiment 1. This may be because the thickness of the eggshell layer is too thick and deeper active species Rh is unable to come into full contact with a reaction substrate. To sum up, appropriate thickness of the eggshell layer can ensure high n-butyraldehyde selectivity (the ratio of n-butyraldehyde to isobutyraldehyde is greater than 8), while ensuring high activity of propylene hydroformylation reaction (except for non-Rh metals, the TOF value of propylene is basically above 700 h⁻¹). In reference example 6, only the competitive adsorbent is impregnated, and it is found from a test that there is no activity of propylene hydroformylation reaction. This proves that the competitive adsorbent only regulates the distribution of the active metal in the eggshell type catalyst during the preparation of the catalyst, but cannot catalyze the hydroformylation reaction of propylene. The spatial distribution of the active metal components in the resin pellets is effectively regulated through a method of finely regulating the porous channel structure of carrier resin pellets and adding the competitive coordination agent, so that the prepared eggshell type catalyst has the characteristics of high activity in the propylene hydroformylation reaction and good selectivity of n-butyraldehyde in the product aldehyde. The eggshell type catalyst has high mechanical strength of carrier resin pellets (measured as about 40 N/pellet), can exist stably in organic solvents without swelling or powdering, and is suitable for industrial application of propylene hydroformylation.

### Embodiment 13

In embodiment 13, when the eggshell type catalyst is prepared, in the step of impregnating the competitive adsorbent, the synthesis process and conditions of the resin pellets and the eggshell type catalyst are the same as those of embodiment 1, except that 0.6 mol•L⁻¹ MgSO₄ aqueous solution is replaced with 0.6 mol•L⁻¹ MgCl₂, AlCl₃, CaCl₂, CuSO₄,ZnSO₄, NaCl, KCl, NaNO₃ and NaHCO₃. The performance results of propylene hydroformylation are shown in Table 2 by the evaluation method of embodiment 12:

**Table 2. Performance of Propylene Hydroformylation of Slurry Bed of Monophosphine Ligand Catalysts Prepared by Different Competitive Adsorbents**

| Entry | Competitive Adsorbent | S_{BET}/(m^{2/}g) | Pore Size Distribution (nm) | Eggshell Layer Thickness (mm) | Propylene TOF(h⁻¹) | Aldehyde Selectivity /% | n-butyraldehyde: isobutyraldehyde |
|---|---|---|---|---|---|---|---|
| 1 | MgSO₄ | 902 | 0.2-10.0 | 0.15 | 733.6 | 99.0 | 8.7 |
| 2 | MgCl₂ | 900 | 0.2-10.0 | 0.10 | 766.9 | 99.1 | 3.2 |
| 3 | AlCl₃ | 901 | 0.2-10.0 | 0.20 | 695.5 | 99.1 | 13.5 |
| 4 | CaCl₂ | 905 | 0.2-10.0 | 0.12 | 754.8 | 98.9 | 5.8 |
| 5 | CuSO₂ | 899 | 0.2-10.0 | 0.19 | 705.7 | 99.5 | 9.4 |
| 6 | ZnSO₂ | 904 | 0.2-10.0 | 0.18 | 724.9 | 99.2 | 9.8 |
| 7 | NaCl | 900 | 0.2-10.0 | 0.02 | 615.8 | 98.5 | 2.1 |
| 8 | KCl | 898 | 0.2-10.0 | 0.02 | 564.1 | 98.3 | 2.2 |
| 9 | NaNO₃ | 901 | 0.2-10.0 | 0.02 | 256.8 | 98.0 | 2.4 |
| 10 | NaHCO₃ | 899 | 0.2-10.0 | 0.02 | 189.5 | 97.3 | 2.3 |

It can be seen from Table 2 that the thickness of the eggshell type catalyst can be significantly changed by using different competitive adsorbents of MgCl₂, AlCl₃, CaCl₂, CuSO₄ and ZnSO₄. On the premise of ensuring a high TOF value of propylene (>690 h⁻¹), the ratio of n-butyraldehyde to isobutyraldehyde is significantly changed. The ratio of n-butyraldehyde to isobutyraldehyde is as high as 13.5 and as low as 3.2, which could not be achieved by using only monophosphine ligand for preparation of the catalyst in the early stage.

Salts of monovalent metal of NaCl, KCl, NaNO₃ and NaHCO₃ are used as the competitive adsorbents, which cannot adjust the thickness of the eggshell layer. The ratio of n-butyraldehyde to isobutyraldehyde is similar to that of reference example 3 without adding the competitive adsorbents. Most crucially, when the anion is NO₃⁻ or HCO₃⁻, the activity of the catalyst may be affected and the TOF value of propylene is only about 200 h⁻¹.

### Embodiment 14

In embodiment 14, when the eggshell type catalyst is prepared, in the step of impregnating the competitive adsorbent, the synthesis process and conditions of the resin pellets and the eggshell type catalyst are the same as those of embodiment 5, except that 0.6 mol•L⁻¹ MgSO₄ aqueous solution is replaced with 0.6 mol•L⁻¹ MgCl₂, AlCl₃, CaCl₂, CuSO₄, ZnSO₄, NaCl, KCl, NaNO₃ and NaHCO₃. The performance results of propylene hydroformylation are shown in Table 3 by the evaluation method of embodiment 12:

**Table 3. Performance of Propylene Hydroformylation of Slurry Bed of Monophosphine/Diphosphine Copolymerization Catalysts Prepared by Different Competitive Adsorbents**

| Entry | Competitive Adsorbent | S_{BET}(m^{2/} g) | Pore Size Distribution (nm) | Eggshell Layer Thickness (mm) | Propylene TOF(h⁻¹) | Aldehyde Selectivity /% | n-butyraldehyde: isobutyraldehyde |
|---|---|---|---|---|---|---|---|
| 1 | MgSO₄ | 897 | 0.2-10.0 | 0.15 | 1236.7 | 98.8 | 29.7 |
| 2 | MgCl₂ | 895 | 0.2-10.0 | 0.10 | 1524.7 | 99.2 | 17.3 |
| 3 | AlCl₃ | 891 | 0.2-10.0 | 0.20 | 1126.8 | 99.1 | 42.5 |
| 4 | CaCl₂ | 898 | 0.2-10.0 | 0.13 | 1446.0 | 99.3 | 18.7 |
| 5 | CuSO₂ | 903 | 0.2-10.0 | 0.20 | 1098.9 | 98.9 | 39.2 |
| 6 | ZnSO₂ | 900 | 0.2-10.0 | 0.20 | 1132.2 | 98.8 | 41.8 |
| 7 | NaCl | 902 | 0.2-10.0 | 0.02 | 1008.1 | 98.7 | 13.2 |
| 8 | KCl | 901 | 0.2-10.0 | 0.02 | 966.41 | 98.4 | 12.5 |
| 9 | NaNO₃ | 907 | 0.2-10.0 | 0.02 | 323.4 | 98.3 | 11.1 |
| 10 | NaHCO₃ | 889 | 0.2-10.0 | 0.02 | 297.1 | 96.8 | 12.8 |

It can be seen from Table 3 that the thickness of the monophosphine/diphosphine copolymerization eggshell type catalyst can be significantly changed by using competitive adsorbents of MgCl₂, AlCl₃, CaCl₂, CuSO₄ and ZnSO₄. The TOF value of propylene can be regulated in the range of 1127-1525 h⁻¹, and the ratio of n-butyraldehyde to isobutyraldehyde can also be regulated in a large range. The ratio of n-butyraldehyde to isobutyraldehyde is as high as 42.5 and as low as 3.2.

Salts of monovalent metal of NaCl, KCl, NaNO₃ and NaHCO₃ are used as the competitive adsorbents, which cannot adjust the thickness of the eggshell layer. The ratio of n-butyraldehyde to isobutyraldehyde is much smaller than the results of salts of bivalent and trivalent metals of MgCl₂, AlCl₃, CaCl₂, CuSO₄ and ZnSO₄. Most crucially, when the anion is NO₃⁻ or HCO₃⁻, the activity of the catalyst may be affected and the TOF value of propylene is only about 300 h⁻¹.

### Embodiment 15

In embodiment 15, when the eggshell type catalyst is prepared, the synthesis process and conditions of the resin pellets and the eggshell type catalyst are the same as those of embodiment 1, except that the monodentate ligand L9 is replaced with other monodentate ligands/polydentate ligands mentioned in claims for autopolymerization. The performance results of propylene hydroformylation are shown in Table 4 by the evaluation method of embodiment 12:

**Table 4. Performance of Propylene Hydroformylation of Slurry Bed of Autopolymerization Eggshell Type Catalysts Prepared by Different Ligands**

| Entry | Ligand | S_{BET}/(m^{2/}g) | Pore Size Distribution (nm) | Eggshell Layer Thickness (mm) | Propylene TOF(h⁻¹) | Aldehyde Selectivity /% | n-butyraldehyde: isobutyraldehyde |
|---|---|---|---|---|---|---|---|
| 1 | L1 | 1109 | 0.2-10.0 | 0.15 | 974.2 | 99.3 | 7.0 |
| 2 | L2 | 768 | 0.2-10.0 | 0.15 | 655.8 | 97.8 | 8.2 |
| 3 | L3 | 799 | 0.2-10.0 | 0.15 | 856.7 | 98.6 | 6.5 |
| 4 | L4 | 783 | 0.2-10.0 | 0.15 | 887.9 | 98.5 | 6.1 |
| 5 | L5 | 568 | 0.2-10.0 | 0.15 | 900.8 | 98.6 | 5.4 |
| 6 | L6 | 765 | 0.2-10.0 | 0.13 | 1347.9 | 98.8 | 4.2 |
| 7 | L7 | 557 | 0.1-0.8 | 0.12 | 1237.3 | 97.9 | 3.9 |
| 8 | L8 | 346 | 0.1-0.8 | 0.12 | 900.2 | 97.9 | 4.3 |
| 9 | L9 | 832 | 0.2-10.0 | 0.15 | 733.6 | 99.0 | 8.7 |
| 10 | L10 | 175 | 0.1-0.5 | 0.17 | 382.0 | 98.5 | 11.5 |
| 11 | L11 | 645 | 0.1-0.6 | 0.16 | 525.8 | 98.9 | 12.4 |
| 12 | L12 | 548 | 0.1-0.8 | 0.14 | 899.6 | 98.8 | 10.0 |
| 13 | BL1 | 89.8 | 0.1-0.5 | 0.17 | 459.0 | 99.4 | 42.5 |
| 14 | BL2 | 85.3 | 0.1-0.5 | 0.20 | 430.1 | 99.2 | 37.6 |
| 15 | BL3 | 80.1 | 0.1-0.5 | 0.18 | 397.4 | 99.3 | 30.1 |
| 16 | BL8 | 90.4 | 0.1-0.5 | 0.18 | 444.4 | 99.5 | 18.9 |
| 17 | BL10 | 78.9 | 0.1-0.5 | 0.19 | 378.9 | 99.5 | 23.2 |
| 18 | BL12 | 538 | 0.1-0.8 | 0.19 | 589.3 | 99.3 | 23.5 |
| 19 | BL13 | 80.2 | 0.1-0.5 | 0.20 | 408.7 | 99.1 | 25.6 |
| 20 | BL14 | 504 | 0.1-0.6 | 0.19 | 833.2 | 99.0 | 21.4 |
| 21 | BL15 | 553 | 0.1-0.8 | 0.18 | 600.2 | 99.4 | 30.2 |
| 22 | BL16 | 562 | 0.1-0.8 | 0.20 | 589.1 | 99.6 | 28.7 |
| 23 | BL17 | 134 | 0.1-0.5 | 0.17 | 405.6 | 99.7 | 30.7 |
| 24 | BL18 | 564 | 0.1-0.6 | 0.18 | 677.9 | 99.6 | 45.8 |

It can be seen from Table 4 that the ratio of n-butyraldehyde to isobutyraldehyde in the eggshell type catalysts prepared by autopolymerization of different monophosphine ligands ranges from 4 to 15. When the same monomer is used, the eggshell type catalyst prepared has higher ratio of n-butyraldehyde to isobutyraldehyde than the catalyst with the same ligand prepared by the previous patent CN202110589670.1. The eggshell type catalysts prepared by autopolymerization of diphosphine ligands BL12, BL14, BL15, BL16 and BL18 containing 4 vinyls have TOF values in the range of 589-833 h⁻¹ and ratios of n-butyraldehyde to isobutyraldehyde in the range of 21-45. Other eggshell type catalysts prepared by bisphosphine ligands (BL1-BL11, BL13 and BL17) with two vinyls have higher ratios of n-butyraldehyde to isobutyraldehyde (23-42), but lower specific surface area and less desirable activity (TOF range is 378-459 h⁻¹). These ligands are suitable for the preparation of high performance eggshell type catalysts in the solution of copolymerization with monophosphine ligands (embodiments 5 and 6, with TOF range of 724-1236 h⁻¹, and the range of the ratios of n-butyraldehyde to isobutyraldehyde of 29-33).

### Reference example 7

In reference example 7, when the eggshell type catalyst is prepared, the synthesis process and conditions of the resin pellets and the eggshell type catalyst are the same as those of embodiment 1, except that Rh(CO)₂(acac) is replaced with Pt(acac)₂ of the same molar number. An eggshell type Pt-based catalyst can be obtained, and the thickness of an eggshell layer is 0.15 mm.

### Reference example 8

In reference example 8, the synthesis process and conditions of the resin pellets and the eggshell type catalyst are the same as those of embodiment 1, except that Rh(CO)₂(acac) is replaced with Fe(acac)₃ of the same molar number. An eggshell type Fe-based catalyst can be obtained, and the thickness of an eggshell layer is 0.15 mm.

### Embodiment 16

1 g of the prepared eggshell type catalysts (the Rh eggshell type catalyst in embodiment 1, the Co eggshell type catalyst in embodiment 8, the Ir eggshell type catalyst in embodiment 9, the Ru eggshell type catalyst in embodiment 10, the Pt eggshell type catalyst in reference example 7, and the Fe eggshell type catalyst in reference example 8) of different metals are loaded into a slurry bed reactor with a capacity of 100 ml, and 60 ml of valeraldehyde is added as slurry liquid. The reaction mixed gas (H₂:CO:C₃H₆=1:1:1) is introduced. The hydroformylation reaction is carried out at 378K, 0.8 MPa, an airspeed of the reaction mixed gas of 1000 h⁻¹ and a stirring rate of 600 RPM. In the reaction, a collection tank at constant temperature of -10°C is used for collection. The reaction products and the slurry liquid carried by tail gas are all dissolved in the collection tank. The collected liquid is analyzed by HP-7890N gas chromatography with HP-5 capillary column and FID, and ethanol is used as the internal standard. The tail gas is analyzed online by HP-7890N gas chromatography with Porapak-QS column and TCD. Reaction results are listed in Table 5.

**Table 5. Performance of Propylene Hydroformylation of Slurry Bed of Eggshell Type Catalysts of Different Active Metals**

| Entry | Catalysts | S_{BET}/(m^{2/} g) | Pore Size Distribution (nm) | Propylene TOF(h⁻¹) | Aldehyde Selectivity /% | n-butyraldehyde: isobutyraldehyde |
|---|---|---|---|---|---|---|
| 1 | Rh in embodiment 1 | 902 | 0.2-10.0 | 733.6 | 99.0 | 8.7 |
| 2 | Co in embodiment 8 | 904 | 0.2-10.0 | 111.6 | 97.4 | 8.4 |
| 3 | Ir in embodiment 9 | 902 | 0.2-10.0 | 63.3 | 98.0 | 8.1 |
| 4 | Ru in embodiment 10 | 903 | 0.2-10.0 | 74.2 | 97.4 | 8.0 |
| 5 | Pt in reference example 7 | 905 | 0.2-10.0 | 60.4 | 98.2 | 2.7 |
| 6 | Fe in reference example 8 | 902 | 0.2-10.0 | 34.2 | 97.3 | 4.2 |

It can be seen from Table 5 that when the active metals are Rh, Co, Ir and Ru, the ratio of n-butyraldehyde to isobutyraldehyde can basically be above 8, while the ratios of n-butyraldehyde to isobutyraldehyde for Pt and Fe are only 2.7 and 4.2, which is not a preferred solution of the patent.

### Embodiment 17

In embodiment 17, the implementation process is the same as that of embodiment 16 except that the reaction condition is adjusted to 418K and 1 Mpa. The reaction under the condition is measured, and the results are listed in Table 6.

**Table 6. Performance of Propylene Hydroformylation of Slurry Bed of Eggshell Type Catalysts of Different Active Metals at 418K and 1 MPa**

| Entry | Catalysts | S_{BET}/(m^{2/} g) | Pore Size Distribution (nm) | Propylene TOF(h⁻¹) | Aldehyde Selectivity /% | n-butyraldehyde: isobutyraldehyde |
|---|---|---|---|---|---|---|
| 1 | Rh in embodiment 1 | 902 | 0.2-10.0 | 1634.2 | 98.8 | 8.6 |
| 2 | Co in | 904 | 0.2-10.0 | 659.1 | 97.1 | 8.2 |
| | embodiment 8 | | | | | |
| 3 | Ir in embodiment 9 | 902 | 0.2-10.0 | 611.3 | 97.8 | 7.9 |
| 4 | Ru in embodiment 10 | 903 | 0.2-10.0 | 701.4 | 97.1 | 7.7 |
| 5 | Pt in reference example 7 | 905 | 0.2-10.0 | 346.2 | 97.0 | 2.2 |
| 6 | Fe in reference example 8 | 902 | 0.2-10.0 | 289.5 | 96.1 | 3.8 |

It can be seen from Table 6 that after the reaction temperature is increased to 418K and the pressure is increased to 1 MPa, the TOF value of the Rh eggshell type catalyst can reach 1634, and the ratio of n-butyraldehyde to isobutyraldehyde is still maintained at a high level. The TOF values (TOF values range from 611 to 701 h⁻¹) of Co, Ir and Ru eggshell type catalysts are also increased significantly. The TOF values of Pt and Fe eggshell type catalysts are only increased to about 300 h⁻¹, which is not a preferred solution of the patent.

### Embodiment 18

1 g of the catalysts prepared by preferred embodiments 1, 2, 5 and 6 of the patent are loaded into a fixed bed reactor respectively, and both ends of the bed layers of the catalysts are loaded with quartz sand. The reaction mixed gas (H₂:CO:C₃H₆=1:1:1, V/V/V) is introduced. The hydroformylation reaction is carried out at 378K, 0.8 MPa, and an airspeed of the reaction mixed gas of 1000 h⁻¹. In the reaction, a collection tank containing 100 ml of cooled deionized water is used for absorption and collection. The reaction product butyraldehyde is fully dissolved in the water in the collection tank. The obtained aqueous solution is analyzed by HP-7890N gas chromatography with HP-5 capillary column and FID, and ethanol is used as the internal standard. After absorption by the water, the reaction tail gas is analyzed online by HP-7890N gas chromatography with Porapak-QS column and TCD. Reaction results are listed in Table 7.

**Table 7. Performance of Propylene Hydroformylation of Fixed Bed of Catalysts in Embodiments 1, 2, 5 and 6**

| Entry | Catalysts | S_{BET}/(m^{2/}g) | Pore Size Distribution (nm) | Propylene TOF(h⁻¹) | Aldehyde Selectivity /% | n-butyraldehyde: isobutyraldehyde |
|---|---|---|---|---|---|---|
| 1 | Embodiment 1 | 902 | 0.2-10.0 | 670.5 | 99.1 | 10.0 |
| 2 | Embodiment 2 | 873 | 0.1-8.0 | 688.4 | 98.9 | 1 12.8 |
| 3 | Embodiment 5 | 897 | 0.1-10.0 | 1200.1 | 99.0 | 3 33.1 |
| 4 | Embodiment 6 | 840 | 0.1-9.0 | 690.2 | 99.2 | 3 36.2 |

It can be seen from Table 7 that when the reactor situation is a fixed bed reactor, the eggshell type catalyst exhibits better performance, and compared with the slurry bed reactor, the ratio of n-butyraldehyde to isobutyraldehyde is significantly increased (which is more than 10 for the eggshell type catalyst prepared by autopolymerization of monophosphine ligands, and more than 33 for the eggshell type catalyst prepared by monophosphine and diphosphine copolymerization). The selectivity of the product aldehyde is slightly increased, while the TOF of propylene is slightly decreased. The reactor form can be flexibly selected according to the requirements for the product.

## Claims

1. An eggshell type catalyst, **characterized in that**: active components of the eggshell type catalyst are selected from one, two or more than two of Rh, Co, Ir, Ru and Pd; a carrier of the eggshell type catalyst is a phosphine ligand resin pellet;
the phosphine ligand resin pellet is formed by autopolymerization of one or more than one of monodentate ligands of vinyl or copolymerization of monodentate ligands of vinyl and polydentate ligands of vinyl; the particle size of the resin pellet ranges from 0.5-7 mm (preferably 0.5-4 mm); metal active components are coordinated on a surface layer of the polymer pellet to form a catalyst eggshell layer; and the thickness (the depth of the metal activity from a carrier surface coordinated to the carrier) of the catalyst eggshell layer (a surface layer of the carrier containing the metal active components is called the eggshell layer) is 0.1-0.2 mm (preferably 0.1-0.15 mm).

2. The eggshell type catalyst according to claim 1, **characterized in that**:
the range of metal load of the eggshell type catalyst is 0.01-8 wt%, and a preferred range is 0.1-2 wt%;
the preparation of the eggshell type catalyst uses a method of excessive solution impregnation; the phosphine ligand resin pellet is added to a solution containing one or more than one of precursors of the active components Rh, Co, Ir, Ru and Pd, stirred fully and coordinated; and active metal of the eggshell type catalyst is coordinated with the phosphine ligand in a resin skeleton to form the catalyst eggshell layer, thereby obtaining the eggshell type catalyst.

3. The eggshell type catalyst according to claim 1 or 2, **characterized in that**: the monodentate ligands of vinyl are one or more than one of the following: 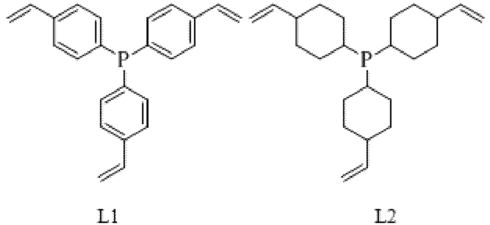 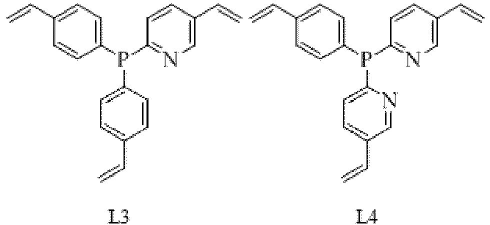 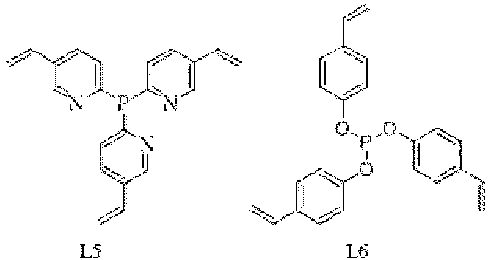 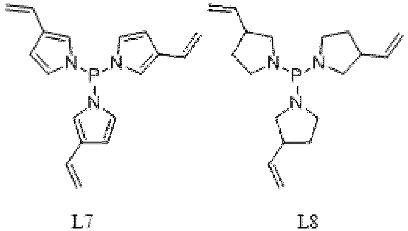 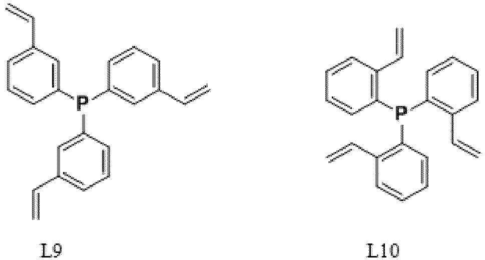 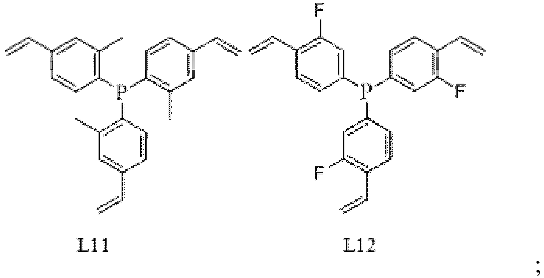 the polydentate ligands of vinyl are one or more than one of the following:

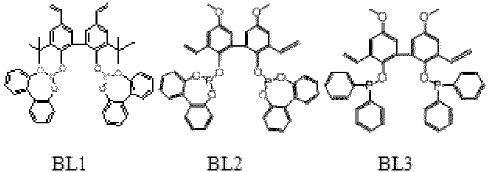

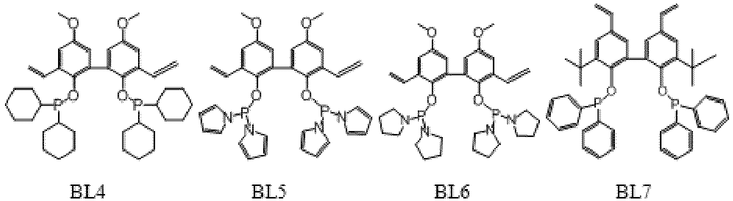

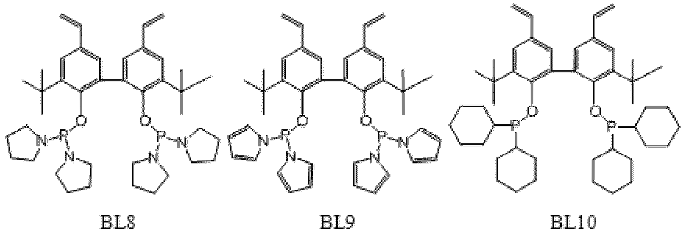

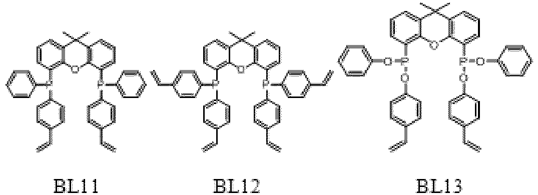

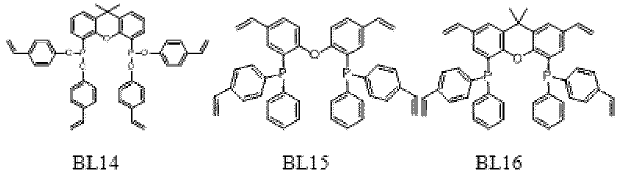

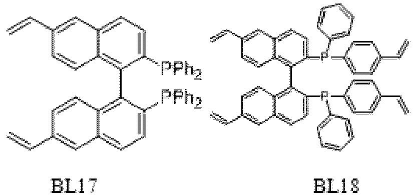

4. The eggshell type catalyst according to any one of claims 1-3, **characterized in that**: the specific surface area of the phosphine ligand resin pellet is 50-3000 m²/g, and a preferred range is 200-1500 m²/g; the pore volume is 0.2-10.0 cm³/g, and preferably 0.5-3.0 cm³/g; and the pore size is distributed in the range of 0.01-100.0 nm, and preferably 0.2-10.0 nm.

5. A preparation method of the eggshell type catalyst according to any one of claims 1-4, **characterized in that**:
a preparation method of the phosphine ligand resin pellet comprises:
after fully dissolving one or more than one of monodentate phosphine ligands of vinyl, adding or not adding polydentate phosphine ligands of vinyl, adding a free radical initiator and preparing the phosphine ligand resin pellet by suspension polymerization;
the preparation method of the eggshell type catalyst comprises: firstly, adding the phosphine ligand resin pellet to the solution containing a competitive coordination agent and then removing a solvent; then adding the phosphine ligand resin pellet to a precursor impregnation solution containing active metal components; fully stirring to coordinate the active metal components with the phosphine ligand in the surface layer of the resin pellet;
and evaporating the solvent to obtain the eggshell type catalyst.

6. The preparation method of the eggshell type catalyst according to claim 5, **characterized in that**:
specific preparation steps of the eggshell type catalyst are:
a) under the protection of inert atmosphere gas of 273-493 K (preferably 273-453 K), adding the monodentate phosphine ligands of vinyl to the solvent, adding or not adding polydentate phosphine ligands of vinyl, and stirring evenly for use to obtain a mixed solution;
b) under the protection of inert atmosphere gas of 298-433 K (preferably 298-393 K), adding a suspending agent to water to fully dissolve, to obtain an aqueous phase; adding the above mixed solution to the aqueous phase while stirring; adding the free radical initiator; and polymerizing the phosphine ligand into a resin pellet at a stirring speed of 20-2000 r/min (preferably 50-800 r/min);
c) taking out solid particles obtained in step b); cleaning by a solvent of 10-1000 (preferably 20-800) times the volume; and then removing the solvent at 298-453 K (preferably 298-393 K) to obtain the phosphine ligand resin pellet;
d) under the protection of inert atmosphere gas of 273-453 K (preferably 273-413 K), stirring the phosphine ligand resin pellet obtained in step c) in a solution containing competitive adsorption coordination for 0.1-10 hours (preferably 0.1-5 hours); then removing the solvent at 298-433 K (preferably 298-393 K); then adding the resin pellet to a solution containing the precursors of the active metal components; stirring for 0.1-100 hours, and preferably for a range of 0.5-12 hours; and next, removing the solvent at 273-433 K (preferably 273-393 K) to obtain the eggshell type catalyst, wherein the concentration range of the competitive adsorption coordination and the active metal in the precursor solution is 0.0001-5 mol•L⁻¹ respectively (preferably 0.0001-1 mol•L⁻¹).

7. The preparation method of the eggshell type catalyst according to claim 6, **characterized in that**:
an organic solvent in step a) is selected from one or more than one of toluene, xylene, benzene, n-alkane of C₅-C₁₅ and cyclohexane;
before the resin pellet is polymerized, the concentration range of the monodentate ligands of vinyl in the solvent is 0.01-1000 g/L, preferably 1-100 g/L; a molar ratio of the monodentate ligands to the polydentate ligands is 5:1-200:1 when the monodentate ligands of vinyl and the polydentate ligands of vinyl are added at the same time; a molar ratio of the monodentate phosphine ligands containing alkylene to the free radical initiator is 500:1-10:1, preferably 100:1-10:1;
the free radical initiator in step b) is one or more than one of dibenzoyl peroxide, cyclohexanone peroxide, tert-butyl hydroperoxide or azodiisobutyronitrile; the suspending agent is selected from one or more than one of N-dodecyl dimethylamine, sodium stearate, calcium dodecylbenzene sulfonate, polyvinyl alcohol and polyvinylpyrrolidone; and a volume ratio of oil phase and aqueous phase in suspension polymerization is 1000:1-1:1000 (preferably 100:1-1:1000);
the solvent for cleaning the resin pellet in step c) is one or more than one of benzene, toluene, xylene, isopropanol, ethanol, dichloromethane, petroleum ether, trichloromethane, water or tetrahydrofuran;
the competitive adsorbent and coordination agent in step d) is selected from one or more than one of MgCl₂, MgSO₄, AlCl₃, CaCl₂, CuSO₄ and ZnSO₄; the impregnation of a competitive adsorbent and the active metal uses a method of excessive solution impregnation; a volume ratio of the resin pellet to an impregnation solution is 1:1.01-1:1000 (preferably 1:1.01-1:500);
the inert atmosphere gas in steps a), b) and d) are selected from one or more than one of CO₂, Ar, He and N₂.

8. The preparation method according to claim 5 or 6, **characterized in that**: the active components are one or more than one of Rh, Co, Ir, Ru and Pd, wherein the precursor of Rh is one or more than one of RhH(CO)(PPh₃)₃, Rh(CO)₂(acac), RhCl₃ and Rh(CH₃COO)₂; the precursor of Co is one or more than one of Co(CH₃COO)₂, Co(CO)₂(acac), Co(acac)₂ and CoCl₂; the precursor of Ir is one or more than one of Ir(CO)₃(acac), Ir(CH₃COO)₃, Ir(acac)₃ and IrCl₄; the precursors of Ru are dichloride (1,5-cyclooctadiene) ruthenium (II), RuCl₃, Ru(acac)₃, triruthenium dodecacarbonyl, [RuAr₂(benzene)]₂, [RuAr₂(p-cymene)]₂, [RuAr₂(mesitylene)]₂, [(π-ally)Ru(cod)]₂ and [(π-ally)Ru(nbd)]₂; the precursor of Pd is one or more than one of Pd(CH₃COO)₂, Pd(acac)₂, PdCl₂, Pd(PPh₃)₄ and PdCl₂(CH₃CN)₂, and the range of the metal load in the catalyst is 0.01-8 wt%, preferably 0.1-2 wt%.

9. An application of the eggshell type catalyst according to any one of claims 1-4 in propylene hydroformylation reaction, comprising a reaction process of loading an eggshell type into a reactor and introducing raw material propylene and mixed gas containing H₂ and CO for propylene hydroformylation reaction.

10. The application according to claim 9, **characterized in that**: the main components of the mixed gas are H₂ and CO; the volume content of H₂+CO is 10-100% (preferably 30-100%); a volume ratio of H_{2/}CO is 0.1-10.0 (preferably 0.6-1.5); other gas components are one or more than one of CO₂, N₂, He and Ar; the purity of the raw propylene is 10-100% (preferably 50-100%), and other gas components are one or more than one of CO₂, N₂, He and Ar; reaction temperature is 313-433 K (preferably 343-393 K); reaction pressure is 0.1-6.0 MPa (preferably 0.3-2.0 MPa); airspeed of the mixed gas is 100-10000 h⁻¹ (preferably 300-3000 h⁻¹); propylene airspeed is 0.01-40.0 h⁻¹ (preferably 0.1-10.0);
the reactor is a slurry bed, a trickle bed, a tank reactor, a radial reactor or a tubular reactor, preferably the slurry bed reactor; the eggshell type catalyst has high activity; the proportion of n-butyraldehyde in the product aldehyde is high; and this type of eggshell type catalyst exhibits a strong prospect of industrial application.
